(19) Europäisches Patentamt
European Patent Office
Office européen des brevets

(11) Veröffentlichungsnummer: **0 255 704**
**A2**

# (12) EUROPÄISCHE PATENTANMELDUNG

(21) Anmeldenummer: **87111084.7**

(22) Anmeldetag: **31.07.87**

(51) Int. Cl.⁴: **C07D 401/14**, C07D 401/04, C07D 417/14, A61K 31/50

(30) Priorität: **08.08.86 DE 3626865**

(43) Veröffentlichungstag der Anmeldung: **10.02.88 Patentblatt 88/06**

(84) Benannte Vertragsstaaten: **AT BE CH DE ES FR GB GR IT LI LU NL SE**

(71) Anmelder: **Boehringer Mannheim GmbH**
**Sandhofer Strasse 116**
**D-6800 Mannheim 31(DE)**

(72) Erfinder: **Mertens, Alfred, Dr. rer. nat.**
**Beethovenstrasse 20**
**D-6905 Schriesheim(DE)**
Erfinder: **von der Saal, Wolfgang, Dr. rer. nat.**
**Neuer Burgweg 3**
**D-6940 Weinheim(DE)**
Erfinder: **Müller-Beckmann, Bernd, Dr. med. vet.**
**Hochgewanne 46**
**D-6718 Grünstadt(DE)**
Erfinder: **Sponer, Gisbert, Dr. med. vet.**
**Lessingstrasse 13**
**D-6941 Laudenbach(DE)**

## (54) Pyridazinon-Derivate, Verfahren zu ihrer Herstellung sowie ihre Verwendung als Arzneimittel.

(57) Pyridazinon-Derivate der Formel I

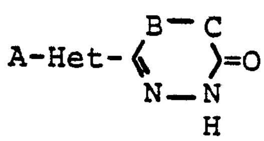

I,

in welcher
Het einen mono-oder bicyclishen Heterocyclus mit ein oder zwei Stickstoffatomen,
B-C eine Gruppe $-CHR_1-CH_2-$, $-CR_1=CH-$, $-CHR_1-O-$, $-O-CHR_1-$, $-CHR_1-NH-$oder $-NH-CHR_1-$, wobei $R_1$ ein Wasserstoffatom oder eine Alkylgruppe bedeutet, und
A eine Amino-, Alkylcarbonylamino-, Aminocarbonylamino-, Aminothiocarbonylamino-, Alkylaminothiocarbonylamino-, Alkylaminocarbonylamino-, N'-Cyano-guanidino-oder N'-Cyano-N''-alkyl-guanidinogruppe, ein ueber ein Stickstoff-oder Schwefelatom gebundener, substituierter Phenylring oder Heterocyclus, oder einen mono-oder bicyclischen, substituierten Heterocyclus mit 1-3 Stickstoffatomen, wobei ein Ringstickstoff direkt an Het gebunden ist, darstellen sowie deren physiologisch verträgliche Salze, Verfahren zu ihrer Herstellung sowie Arzneimittel, die diese Verbindungen enthalten zur Behandlung von Herz-und Kreislauferkrankungen.

EP 0 255 704 A2

**Neue Pyridazinon-Derivate, Verfahren zu ihrer Herstellung sowie ihre Verwendung als Arzneimittel**

Die vorliegende Erfindung betrifft neue Pyridazinon-Derivate der allgemeinen Formel I.

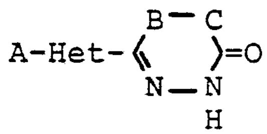 I,

in welcher
Het einen mono-oder bicyclischen Heterocyclus mit ein oder zwei Stickstoffatomen,
B-C eine Gruppe $-CHR_1-CH_2-$, $-CR_1=CH-$,$-CHR_1-O-$, $-O-CHR_1-$, $-CHR_1-NH-$oder $-NH-CHR_1-$, wobei $R_1$ ein Wassersoffatom oder eine Alkylgruppe bedeutet, und
A eine Amino-, Alkylcarbonylamino-, Aminocarbonylamino-, Aminothiocarbonylamino-, Alkylaminothiocarbonylamino-, Alkylaminocarbonylamino-, N'-Cyano-guanidino-oder N'-Cyano-N''-alkyl-guanidinogruppe, ein ueber ein Stickstoff-oder Schwefelatom gebundener, substituierter Phenylring oder Heterocyclus, oder einen mono-oder bicyclischen, substituierten Heterocyclus mit 1-3 Stickstoffatomen, wobei ein Ringstickstoff direkt an Het gebunden ist, darstellen sowie deren physiologisch verträgliche Salze, Verfahren zu ihrer Herstellung sowie Arzneimittel, die diese Verbindungen enthalten.

Fuer den Fall, daß die Verbindungen der allgemeinen Formel I ein asymmetrisches Kohlenstoffatom besitzen, sind auch Gegenstand der Erfindung die optisch aktiven Formen und racemischen Gemische dieser Verbindungen.

Diese neuen Verbindungen der vorliegenden Erfindung weisen wertvolle pharmakologische Eigenschaften auf, insbesondere steigern sie die Herzkraft und/oder wirken blutdrucksenkend und/oder beeinflussen die Thrombozytenaggregation und verbessern die Mikrozirkulation.

In der allgemeinen Formel I stellt Het einen mono-oder bicyclischen Heterocyclus mit ein oder zwei Stickstoffatomen dar, bevorzugt in diesem Sinne sind 6-gliedrige Heterocyclen, wie Pyridin-, Pyrazin-, Pyrimidin-, Pyridazinrest. Bevorzugte Bicyclen sind der Chinolin-, Isochinolin-, Chinoxalin-, Chinazolin-und Phthalazinrest.

Bedeutet B-C in der allgemeinen Formel I eine Gruppe $-CHR_1-CH_2-$, $-CR_1=CH-$, $-CHR_1-O-$, $-O-CHR_1-$, $-CHR_1-NH-$oder $-NH-CHR_1-$, so sind in diesem Sinne bevorzugt die Gruppen $-CHR_1-CH_2-$, $-CR_1=CH-$, $-CHR_1-O-$und $-CHR_1-NH-$, wobei $R_1$ in den vorgenannten Gruppen ein Wasserstoffatom oder eine Alkylkette mit 1 - 3 C-Atomen darstellen kann.

Bedeutet A in der allgemeinen Formel I eine Amino-, Alkylcarbonylamino-, Aminocarbonylamino-, Aminothiocarbonylamino-, Alkylaminothiocarbonylamino-, Alkylaminocarbonylamino-, N'-Cyano-guanidino-oder N'-Cyano-N''-alkyl-guanidinogruppe, so kommen vorzugsweise die Amino-, Alkylcarbonylamino-, Alkylaminocarbonylamino-oder N'-Cyano-N''-alkyl-guanidinogruppe, wobei die Alkylreste der vorgenannten Gruppen 1-5 C-Atome enthalten koennen, sowie die Aminocarbonylamino-und Aminothiocarbonylaminogruppe in Betracht.

Bedeutet A der allgemeinen Formel I einen ueber ein Stickstoff-oder Schwefelatom gebundenen, substituierten Phenylring oder Heterocyclus, so sind in diesem Sinne bevorzugt der Phenylamino-, Pyridylamino-, Thiazolamino-, Tetrazolamino-, Phenylthio-, Pyridylthio-, Thiazolthio-, Triazolthio-und Tetrazolthiorest, wobei der Phenylring oder die Heterocyclen als Substituenten eine oder mehrere $C_1$-$C_6$ Alkyl-, $C_1$-$C_6$ Alkoxy-, Hydroxy-, Nitro-oder Halgenreste, vorzugsweise Fluor-, Chlor-und Bromreste, tragen koennen.

Bedeutet A in der allgemeinen Formel I einen mono-oder bicyclischen substituierten Heterocyclus mit 1-3 Stickstoffatome, wobei ein Ringstickstoff direkt an Het gebunden ist, so sind in diesem Sinne Fuenfringe mit 1-3 Stickstoffatome, Sechsringe mit 1-3 Stickstoffatomen und bicyclische Hetero cyclen mit 1-2 Stickstoffatomen bevorzugt, wobei die vorgenannten Heterocyclen durch eine oder mehrere Alkyl-, Alkoxy-, Hydroxy-, Alkylcarbonyl-oder Alkylcarbonylaminogruppe substituiert sein können und die zuvor genannten Alkylgruppen 1-6 C-Atome enthalten können.

Besonders bevorzugte Verbindungen der allgemeinen Formel I sind jedoch diejenigen, in denen
Het eine Pyridin-, Pyrazin-, Pyrimidin-, Pyridazin-oder Phthalazinring bedeutet,
B-C eine Gruppe $-CHR_1-CH_2-$, $-CR_1=CH-$, $-CHR_1-O-$oder $-CHR_1-NH-$darstellt, wobei $R_1$ in den vorgenannten Gruppen ein Wasserstoffatom oder die Methylgruppe ist,
und

A eine Alkylcarbonylamino-, Alkylaminocarbonylamino-, oder N'-Cyano-N''-alkyl-guanidinogruppe, wobei die Alkylreste 1-3 C-Atome enthalten, oder eine Aminocarbonylamino-oder Aminothiocarbonylaminogruppe ist, oder

A einen Amino-, Phenylamino-Pyridylamino-, Thiazolamino-, Tetrazolamino-oder Triazolthiorest bedeutet, wobei der Phenylring oder die Heterocyclen durch ein oder mehrere Methyl-, Methoxy-, Hydroxy-oder Chlorreste substituiert sein koennen, oder

A einen monocyclischen Fuenfring mit 1-3 Stickstoffatomen bedeutet, wobei in erster Linie der Pyrrol-, Pyrazol-, Imidazol-, Imidazolin-und Triazolring in Frage kommt, oder A einen monocyclischen Sechsring mit 1-3 Stickstoffatomen darstellt, wobei der Piperidin-, Piperazin-oder Triazinrest in Frage kommt, oder A ein bicyclischer Heterocyclus ist, wobei der Benzimidazol-und 4,5,6,7-Tetrahydrobenzimidazolring besonders bevorzugt ist. Als Substituenten fuer die oben genannten monocyclischen Fuenf-und Sechsringe und die bicyclischen Heterocyclen kommen als besonders bevorzugte Reste eine oder mehrere Methyl-, Hydroxy-, Methylcarbonyl-oder Methylcarbonylaminogruppen in Frage.

Die Herstellung der Verbindungen der allgemeinen Formel I kann in der Weise erfolgen, daß man entweder

a) $\gamma$-Ketocarbonsaeuren der allgemeinen Formel II,

$A\text{-}Het\text{-}CO\text{-}\overset{R_1}{C}H\text{-}CH_2\text{-}COOH$ II,

mit Hydrazin zu Verbindungen der allgemeinen Formel I cyclisiert, in der A, Het und $R_1$ die angegebene Bedeutung haben und B-C die Gruppe $-CHR_1-CH_2-$darstellt, und gegebenenfalls die so erhaltenen Verbindungen der Formel I durch Oxidation zu anderen Verbindungen der Formel I, in der A, Het und $R_1$ die angegebene Bedeutung haben und B-C die Gruppe $-CR_1=CH-$darstellt, umwandelt,

b) Hydrazone der allgemeinen Formel III,

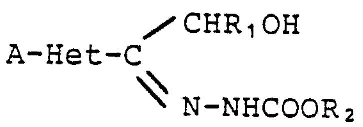

III

in der A, Het und $R_1$ die angegebene Bedeutung haben und $R_2$ ein niederer Alkylrest wie Methyl oder Ethyl ist, zu Verbindungen der allgemeinen Formel I, in der A, Het und $R_1$ die angegebene Bedeutung haben und B-C die Gruppe $-CHR_1-O-$bedeutet, cyclisiert,

c) Hydrazide der allgemeinen Formel IV,

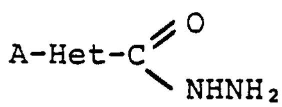

IV,

in denen A und Het die angegebene Bedeutung haben, mit Saeuren der allgemeinen Formel V,

$Hal\text{-}CHR_1\text{-}COOH$ V,

in denen $R_1$ die angegebene Bedeutung hat und Hal ein Halogenatom ist, oder mit reaktiven Derivaten der Saeure zu Verbindungen der allgemeinen Formel I, in der A, Het und $R_1$ die angegebene Bedeutung haben und B-C die Gruppe $-O-CHR_1-$bedeutet, cyclisiert,

d) Iminoether der allgemeinen Formel VI,

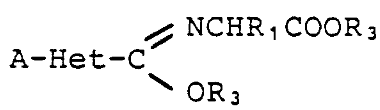

VI,

in denen A, Het und $R_1$ die angegebene Bedeutung haben, und die beiden Reste $R_3$ gleich oder verschieden sind und Alkylreste mit 1-4 C-Atomen bedeuten, mit Hydrazin zu Verbindungen der allgemeinen Formel I, in denen A, Het und $R_1$ die angegebene Bedeutung haben und B-C die Gruppe $-NH-CHR_1-$ bedeutet, cyclisiert,

e) Verbindungen der allgemeinen Formel VII,

A-Het-$COCHR_1NHCOOR_4$ VII,

in denen A, Het und $R_1$ die angegebene Bedeutung haben, und $R_4$ einen Alkylrest mit 1-4 C-Atomen bedeutet, mit Hydrazin zu Verbindungen der allgemeinen Formel I, in denen A, Het und $R_1$ die angegebene Bedeutung haben und B-C die Gruppe $-CHR_1-NH$-bedeutet, cyclisiert.

Die bei den Verfahren a, d und e beschriebenen Umsetzungen von Verbindungen der allgemeinen Formeln II, VI und VII mit Hydrazin werden vorzugsweise in einem Loesungsmittel wie z.B. Wasser oder Alkohol bei Temperaturen zwischen 0°C und dem Siedepunkt des Loesungsmittels durchgefuehrt.

Die im Verfahren a ebenfalls beschriebene Oxidation des 4,5-Dihydro-3(2H)pyridazinons zum -3(2H)-Pyridazinon gelingt durch literaturbekannte Verfahren wie Bromierung/Dehydrobromierung, edelmetallkatalysierte Dehydrierung oder durch Oxidation/Reduktion Verfahren mit $MnO_2$ oder m-Nitrobenzolsulfonsaeure als Reagenzien (J. Med. Chem. 17, 273, 1974).

Die im Verfahren b beschriebene Cyclisierung von Verbindungen der allgemeinen Formel III wird vorteilhaft in Gegenwart einer Base, wie z.B. Natriumhydroxid oder Natriumalkoholat, in einem Loesungsmittel wie Wasser oder Alkohol bei Temperaturen zwischen 0°C und dem Siedepunkt des Loesungsmittels durchgefuehrt.

Die im Verfahren c beschriebene Cyclisierung von Verbindungen der allgemeinen Formel IV mit Holgencarbonsaeuren oder reaktiven Derivaten der Saeure wird vorzugsweise in zwei Stufen durchgefuehrt, wobei zunaechst vorteilhaft ein Halogencarbonsaeurehalogenid mit dem Hydrazid umgesetzt wird. Die Reaktion wird in inerten Loesungsmitteln, wie z.B. Toluol, Methylenchlorid oder Pyridin gegebenenfalls unter Zusatz einer Base, wie Kaliumcarbonat oder Triethylamin, bei Temperaturen zwischen 0°C und 100°C durchgefuehrt. Das so erhaltene Diacylhydrazin wird in der zweiten Stufe mit einer Base, wie z.B. Natriumhydrid oder Alkalicarbonat, in Loesungsmitteln wie DMF oder Aceton bei erhoehter Temperatur zwischen 40°C und 150°C cyclisiert.

Für den Fall, daß nach den oben beschriebenen Verfahren Verbindungen der allgemeinen Formel I erhalten werden, in der A eine Aminogrupe darstellt, können diese Verbindungen anschließend in andere Verbindungen der allgemeinen Formel I umgewandelt werden, in der A die zu Beginn angegebene Definition hat. Diese Umwandlungen geschehen beispielsweise durch Acylierung, oder Addition von Isocyanaten, Isothiocyanaten oder Carbonyldiimiden, wobei Verbindungen der allgemeinen Formel I erhalten werden, in der A eine Alkycarbonylamino-, Aminocarbonylamino-, Aminothiocarbonylamino-, Alkylaminothiocarbonylamino-, Alkylaminocarbonylamion-, N'-Cyanoguanidino-oder N'-Cyano-N''-alkylguanidinogruppe bedeutet.

Ferner koennen die erhaltenen Verbindungen der allgemeinen Formel I anschließend gewuenschtenfalls in ihre physiologisch vertraeglichen Saeureadditionssalze mit anorganischen oder organischen Saeuren ueberfuehrt werden. Als Saeuren kommen hierfuer beispielsweise Salzsaeure, Bromwasserstoffsaeure, Schwefelsaeure, Phosphorsaeure, Fumarsaeure, Bernsteinsaeure, Weinsaeure, Zitronensaeure, Milchsaeure, Maleinsaeure oder Methansulfonsaeure in Betracht.

Wie bereits eingangs erwaehnt weisen die neuen Verbindungen der allgemeinen Formel I, deren Tautomere und deren physiologisch vertraegliche Saeureadditionssalze bei einer langen Wirkungsdauer ueberlegene pharmakologische Eigenschaften auf, insbesondere eine blutdrucksenkende und/oder positivinotrope Wirkung und/oder beeinflussen die Thormbozytenfunktion und verbessern die Mikrozirkulation.

Zur Herstellung von Arzneimitteln werden die Substanzen der allgemeinen Formel I in an sich bekannter Weise mit geeigneten pharmazeutischen Traegersubstanzen, Aroma-, Geschmacks-und Farbstoffen gemischt und beispielsweise als Tabletten oder Dragees ausgeformt oder unter Zugabe entsprechender Hilfsstoffe in Wasser oder Oel, wie z.B. Olivenoel, suspendiert oder geloest.

Die erfindungsgemaeßen neuen Substanzen der allgemeinen Formel I und ihre Salze koennen in fluessiger oder fester Form enteral oder parenteral appliziert werden. Als Injektionsmedium kommt vorzugsweise Wasser zur Anwendung, welches die bei Injektionsloesungen ueblichen Zusaetze wie Stabilisierungsmittel, Loesungsvermittler oder Puffer enthaelt.

Derartige Zusaetze sind z.B. Tartrat-und Citratpuffer, Ethanol, Komplexbildner (wie Ethylendiamintetraessigsaeure und deren nicht toxische Salze) und hochmolekulare Polymere (wie fluessiges Polyethylenoxid) zur Viskositaetsregulierung. Feste Traegerstoffe sind z.B. Staerke, Lactose, Mannit, Methylcellulose, Talkum, hochdisperse Kieselgelsaeuren, hochmolekulare Fettsaeuren (wie Stearinsaeure), Gelatine, Agar-Agar, Calciumphosphat, Magnesiumstearat, tierische und pflanzliche Fette und feste hochmolekulare Polymere (wie Polyethylenglykole). Fuer orale Applikation geeignete Zubereitungen koennen gewuenschtenfalls Geschmacks-und Sueßstoffe enthalten.

Die erfindungsgemaeßen Verbindungen werdenueblicherweise in Mengen von 1 - 50 mg pro Tag bezogen auf 75 kg Koerpergewicht appliziert. Bevorzugt ist es, 2-3 mal pro Tag 1-2 Tabletten mit einem Wirkstoffgehalt von 0.5 - 20 mg zu verabreichen. Die Tabletten koennen retardiert sein, wodurch nur noch 1 mal pro Tag 1-2 Tabletten mit 1 - 50 mg Wirkstoff gegeben werden muß. Der Wirkstoff kann auch durch Injektion 1-8 mal pro Tag bzw. durch Dauerinfusion gegeben werden, wobei Mengen von 0.5 - 20 mg Tag normalerweise ausreichen.

Bevorzugt im Sinne der vorliegenden Erfindung sind außer den in den Beispielen genannten Verbindungen die folgenden und deren Tautomere:

4,5-Dihydro-6-[5-(1H-pyrrol-1-yl)-2-pyridyl]-3(2H)-pyridazinon
4,5-Dihydro-6-[2-(1H-pyrrol-1-yl)-6-pyridyl]-3(2H)-pyridazinon
4,5-Dihydro-3-[2-(1H-imidazol-1-yl)-5-pyridyl]-6(1H)-1,2,4-triazinon
2-[2-(1H-Imidazol-1-yl)-5-pyridyl]-5(4H,6H)-1,3,4-oxadiazinon
4,5-Dihydro-5-methyl-6-[2-(1H-pyrazol-1-yl)-6-pyrimidinyl)-3(2H)-pyridazinon
4,5-Dihydro-5-methyl-6-[3-(1H-pyrazol-1-yl)-6-pyridazinyl)-3(2H)-pyridazinon
4,5-Dihydro-5-methyl-6-[2-(2-oxo-imidazolidin-1-yl)-5-pyridazinyl)-3(2H)-pyridazinon
4,5-Dihydro-6-[2-(1H-1,2,4-triazol-1-yl)-3-pyridyl]-3(2H)-pyridazinon
4,5-Dihydro-6-[2-(4H-1,2,4-triazol-4-yl)-3-pyridyl]-3(2H)-pyridazinon
4,5-Dihydro-5-methyl-6-[2-(1,2,3,4,5,6-hexahydro-2,4,6-trioxo-1,3,5-triazin-1-yl)-4-pyridyl]-3(2H)-pyridazinon
4,5-Dihydro-5-methyl-6-[2-(1H-benzimidazol-1-yl)-5-pyrimidinyl]-3(2H)-pyridazinon
4,5-Dihydro-5-methyl-6-[2-(4,5,6,7-tetrahydro-1H-benzimidazol-1-yl)-5-pyrimidinyl-3(2H)-pyridazinon
4,5-Dihydro-3-[1-(1H-imidazol-1-yl)-4-phthalazinyl]-6(1H)-1,2,4-triazinon
2-[1-(1H-Imidazol-1-yl)-4-phthalazinyl]-5(4H,6H)-1,3,4-oxadiazinon
4,5-Dihydro-6-[2-(acetylamino)-5-pyridyl]-3(2H)-pyridazinon
6-[2-(Aminocarbonylamino)-5-pyridyl]-3(2H)-pyridazinon
5-Methyl-6-[2-(methylaminocarbonylamino)-5-pyridyl]-3(2H)-pyridazinon
4,5-Dihydro-6-[2-(methylaminothiocarbonylamino)-5-pyridyl]-3-(2H)-1,2,4-triazinon
5-[2-(N'-Cyano-N"-methyl-guanidino)-5-pyridyl]-2(3H,6H)-1,3,4-oxadiazinon
4,5-Dihydro-6-[2-(phenylamino)-6-pyridyl]-3(2H)-pyridazinon
4,5-Dihydro-6-[2-(2-thiazolylamino)-6-pyridyl]-3(2H)-pyridazinon
4,5-Dihydro-6-[2-(5-tetrazolylamino))-6-pyridyl]-3(2H)-pyridazinon
4,5-Dihydro-6-[2-(1,2,4-triazol-5-ylthio)-5-pyridyl)-3(2H)-pyridazinon
6-[2-(4-Acetyl-1-piperazinyl)-5-pyridyl]-3(2H)-pyridazinon
4,5-Dihydro-5-methyl-6-[2-(4-acetylamino-1-piperazinyl)-5-pyrimidinyl]-3(2H)-pyridazinon
4,5-Dihydro-5-methyl-6-[2-(aminocarbonylamino)-5-pyridyl]-3(2H)-pyridazinon
4,5-Dihydro-5-methyl-6-[2-(aminothioarbonylamino)-5-pyridyl]-3(2H)-pyridazinon
4,5-Dihydro-5-methyl-6-[2-(N -cyano-N"-methyl-guanidino)-5-pyridyl]-3(2H)-pyridazinon
4,5-Dihydro-5-methyl-6-[2-(2-phenylamino-5-pyridyl]-3(2H)-pyridazinon
4,5-Dihydro-5-methyl-6-[2-(2-thiazolylamino)-5-pyridyl]-3(2H)-pyridazinon
4,5-Dihydro-5-methyl-6-[2-(5-1H-tetrazolylamino)-5-pyridyl]-3(2H)-pyridazinon
4,5-Dihydro-5-methyl-6-[2-(5-1H-tetrazolylthio)-5-pyridyl]-3(2H)-pyridazinon
4,5-Dihydro-5-methyl-6-[2-(4-acetyl-1-piperazinyl)-5-pyridyl]-3(2H)-pyridazinon
4,5-Dihydro-5-methyl-6-[2-(4-acetylamino-1-piperidinyl)-5-pyridyl]-3(2H)-pyridazinon
4,5-Dihydro-5-methyl-6-[2-(1-pyrazolyl)-5-pyridyl]-3(2H)-pyridazinon
4,5-Dihydro-5-methyl-6-[2-(2-oxo-1-imidazolidinyl)-5-pyridyl]-3(2H)-pyridazinon
4,5-Dihydro-5-methyl-6-[2-(4H-1,2,4-triazol-4-yl)-5-pyridyl]-3(2H)-pyridazinon
4,5-Dihydro-5-methyl-6-[2-(1H-imidazol-1-yl)-4-pyridyl]-3(2H)-pyridazinon
4,5-Dihydro-5-methyl-6-[2-(1H-imidazol-1-yl)-6-pyridyl]-3(2H)-pyridazinon
4,5-Dihydro-5-methyl-6-[2-(1H-imidazol-1-yl)-3-pyridyl]-3(2H)-pyridazinon
4,5-Dihydro-5-methyl-6-[2-(1H-imidazol-1-yl)-5-pyrimidinyl]-3(2H)-pyridazinon
5-[2-(1H-Imidazol-1-yl)-5-pyridyl]-2(3H,6H)-1,3,4-oxadiazinon
4,5-Dihydro-5-methyl-6-[2-(1H-imidazol-1-yl)-5-pyridyl]-3(2H)-1,2,4-triazinon

Beispiel 1

4,5-Dihydro-5-methyl-6-[2-(1H-imidazol-1-yl)-5-pyridyl]-3(2H)-pyridazinon

Zu 1.73 g NaH (55proz. Dispersion in Mineraloel) in 130 ml abs. Tetrahydrofuran wurden 10.8 g (40 mmol) 2-[2-(1H-Imidazol-1-yl)-5-pyridyl]-2-(4-morpholino)acetonitril zugegeben und bei 25° C 3.24 ml (40 mmol) Crotonsaeurenitril zugetropft. Nach 2 Stunden wurde zur Trockne abdestilliert, der Rueckstand in Wasser suspendiert und salzsauer (pH 3) 2x mit Dichlormethan extrahiert. Die waessrige Phase wurde ammoniakalisch (pH 8.5) gestellt, 3x mit Dichlormethan extrahiert, die organischen Phasen mit Wasser gewaschen, ueber $Na_2SO_4$ getrocknet und das Loesungsmittel abgedampft. Man erhaelt 12.8 g 2-(4-Morpholino)-2-[2-(1H-imidazol-1-yl)-5-pyridyl]-3-methyl-glutarsaeuredinitril als Rohprodukt.

10.5 g des Rohproduktes wurde mit 55 ml 6 N HCl eine Stunde auf 100°C erhitzt. Der Ansatz wurde im Vakuum zur Trockne destilliert, der Rueckstand mit 200 ml Ethanol erwaermt und abgesaugt. Das ethanolische Filtrat, welches als Rohprodukt 3-Methyl-4-oxo-4-[2-(1H-imidazol-1-yl)-5-pyridyl]buttersaeure enthaelt, wurde mit 15 ml 85proz. Hydrazinhydrat-Loesung versetzt und 2.5 Stunden auf 100°C erhitzt. Der eingedampfte Rueckstand wurde mit Wasser durchgearbeitet, abgesaugt, nochmals in Wasser suspendiert, mit Ammoniak auf pH 7 gestellt und abgesaugt. Nach Umkristallisieren aus Methanol erhaelt man 4.3 g (53 %) der Titelverbindung vom Schmp. 217-19°C.

Das Ausgangsmaterial kann wie folgt hergestellt werden:

a) 12.2 g (180 mmol) Imidazol, 24.75 g (180 mmol) $K_2CO_3$, 20 g (144 mmol) 2-Chlor-5-cyano-pyridin und 350 ml Dimethylsulfoxid werden zusammengegeben und 8 Stunden unter Stickstoff bei 70°C geruehrt. Anschließend wird im Hochvakuum zur Trockne destilliert, der Rueckstand in Wasser suspendiert, mit 2 N Salzsaeure angesaeuert und die Faellung durch Wasser zusatz in Loesung gebracht. Das Filtrat wird 2x mit Dichlormethan ausgeschuettelt, die waessrige Phase von anhaftenden Dichlormethan befreit, mit 2 N Ammoniak auf pH 8.5 gestellt und abgesaugt. Man erhaelt 23 g (93.4 %) 2-(1H-Imidazol-1-yl)-5-cyano-pyridin vom Schmp. 168-70°C.

b) 11.5 g (67.6 mmol) des obigen Produktes, 11.5 g Nickel/Aluminium-Pulver, 41.4 ml 100proz. Ameisensaeure und 15.1 ml Wasser werden zusammengegeben und unter Stickstoff 20 Stunden auf 70°C erwaermt. Anschließend wurde warm abgesaugt, mit warmer 90proz. Ameisensaeure nachgewaschen, das Filtrat unter Eiskuehlung mit Ammoniak neutralisiert und abgesaugt. Man erhaelt 9.6 g (82 %) 2-(1H-Imidazol-1-yl)pyridin-5-aldehyd vom Schmp. 133°C.

c) 9.5 g (54.8 mmol) des obigen Aldehyds wurden mit 10.45 g (54.9 mmol) p-Toluolsulfonsaeure, 6.42 g (73.7 mmol) Morpholin und 57 ml Dioxan vorgelegt. In die Vorlage wurde unter Stickstoff eine Loesung vom 3.61 g (55.4 mmol) Kaliumcyanid in 3.3 ml Wasser zugetropft und anschließend 3.5 Stunden bei 110°C geruehrt. Der Ansatz wurde stark eingeengt, mit gesaettigter $K_2CO_3$-Loesung durchgearbeitet und nach Zusatz von wenig Wasser mit Dichlormethan extrahiert. Die vereinigten organischen Phasen wurden mit Wasser gewaschen, getrocknet und eingeengt. Der Rueckstand wurde aus Dichlormethan/Ether unter Zusatz von Kohle umkristallisiert. Man erhaelt 11.8 g (79.5 %) 2-[2-(1H-Imidazol-1-yl)-5-pyridyl]-2-(4-morpholino)acetonitril vom Schmp. 148 - 50°C.

Beispiel 2

4,5-Dihydro-6-[2-(1H-imidazol-1-yl)-5-pyridyl]-3(2H)-pyridazinon

9.5 g (35.3. mmol) 2-[2-(1H-Imidazol-1-yl)-5-pyridyl]-2-(4-morpholino)acetonitril wurden in 105 ml absolutem Tetrahydrofuran suspendiert, 7.4 ml 30proz. methanolische Kaliumhydroxydlösung zugesetzt und anschließend 2.55 ml (38.5 mmol) Acrylnitril zugetropft. Nach 3 Stunden wurde das THF abdestilliert, der Rückstand mit Wasser und Dichlormethan versetzt, mit Eisessig angesäuert und das Produkt 3 mal mit Dichlormethan extrahiert. Die organische Phase wurde 2 mal mit Wasser gewaschen, getrocknet und eingeengt. Der Rückstand wurde in Dichlormethan gelöst, mit Ether versetzt und die Kristalle abgesaugt. Man erhält 10.3 g 2-(4-Morpholino)-2-[2-(1H-imidazol-1-yl)-5-pyridyl] glutarsäuredinitril vom Schmp. 154-156°C.

10 g (31 mmol) des obigen Dinitrils wurden 1.5 Stunden mit 6 N Salzsäure gekocht, anschließend eingedampft, der Rückstand in Ethanol aufgekocht und nach dem Abkühlen vom Ungelösten abgetrennt. Das Filtrat wurde mit Hydrazin-Hydrat bis zur alkalischen Reaktion versetzt und 3 Stunden am Rückfluß erhitzt. Der eingedampfte Rückstand wurde an Kieselgel (Laufmittel: Dichlormethan/Methanol 96:4) gereinigt. Man erhält 4.2 g der Titelverbindung vom Schmp. 283-85°C.

Beispiel 3

4.5-Dihydro-5-methyl-6-[2-(1H-benzimidazol-1-yl)-5-pyridyl]-3(2H)-pyridazinon

Analog Beispiel 2 erhält man aus 3 g (9.8 mmol) 2-[2-(1H-benzimidazol-1-yl)-5-pyridyl]-2-(4-morpholino)acetonitril und Crotonsäurenitril nach Reinigung an Kieselgel (Laufmittel: Dichlormethan/Methanol 99:1) 2.2 g 2-(4-Morpholino)-2-[2-(1H-benzimidazol-1-yl)-5-pyridyl]-3-methylglutarsäuredinitril als schaumigen Rückstand, woraus nach Hydrolyse, Umestzung mit Hydrazin-Hydrat und Reinigung an Kieselgel (Laufmittel: Dichlormethan/Methanol 95:5) 0.6 g der Titelverbindung vom Schmp. 206-07°C aus Ethanol erhalten werden.

Nach Beispiel 1a - c werden folgende Zwischenprodukte erhalten:

3a) 2-(1H-Benzimidazol-1-yl)-5-cyanopyridin vom Schmp. 193-95°C.

b) 2-(1H-Benzimidazol-1-yl)pyridin-5-aldehyd vom Schmp. 148-150°C.

c) 2-[2-(1H-Benzimidazol-1-yl)-5-pyridyl]-2-(4-morpholino)acetonitril vom Schmp. 138-40°C.

Beispiel 4

4,5-Dihydro-5-methyl-6-[2-(4,5,6,7-tetrahydro-1H-benzimidazol-1-yl)-5-pyridyl]-3(2H)-pyridazinon

Analog Beispiel 2 erhält man aus 1 g (3.10 mmol) 2-[2-(4,-5,6,7-tetrahydro-1H-benzimidazol-1-yl)-5-pyridyl]-2-(4-morpholino)acetonitril und Crotonsäurenitril nach Reinigung an Kieselgel (Laufmittel: Dichlormethan/Methanol 98:2) 0.65 g 2-(4-Morpholino)-2-[2-(4,5,6,7-tetrahydro-1H-benzimidazol-1-yl)-5-pyridyl]-3-methylglutarsäuredinitril vom Schmp. 102-04°C, woraus nach Hydrolyse, Umsetzung mit Hydrazin-Hydrat und Reinigung an Kieselgel (Laufmittel: Dichlormethan/Methanol 97:3) 0.2 g der Titelverbindung vom Schmp. 234-35°C aus Ethanol erhalten werden.

Nach Beispiel 1a - c werden folgende Zwischenproduckte erhalten:

4a) 2-(4,5,6,7-Tetrahydro-1H-benzimidazol-1-yl)-5-cyanopyridin vom Schmp. 197-200°C.

b) 2-(4,5,6,7-Tetrahydro-1H-benzimidazol-1-yl)pyridin-5-aldehyd vom Schmp. 84-88°C.

c) 2-[2-(4,5,6,7-Tetrahydro-1H-benzimidazol-1-yl)-5-pyridyl]-2-(4-morpholino)acetonitril vom Schmp. 141-42°C.

Beispiel 5

6-[2-(1H-Imidazol-1-yl)-5-pyridyl]-3(2H)-pyridazinon

2 g (8.3 mmol) der in Beispiel 2 hergestellten Verbindung wurden in 250 ml Dioxan mit 50 g Braunstein 5 Stunden gekocht, abgesaugt und das Filtrat bis zur Kristallisation eingeengt. Nach Umkristallisieren aus Ethanol erhält man 1 g der Titelverbindung vom Schmp. 300°C.

Analog erhält man aus der in Beispiel 1 hergestellten Verbindung:

a) 5-Methyl-6-[2-(1H-Imidazol-1-yl)-5-pyridyl]-3(2H)-pyridazinon vom Schmp. 261-63°C aus Methanol.

Beispiel 6

4,5-Dihydro-5-methyl-6-[2-(1H-1,2,4-triazol-1-yl)-5-pyridyl]-3(2H)-pyridazinon

Analog Beispiel 2 erhält man aus 21.7 g (66.4 mmol) 2-[2-(1H-1,2,4-triazol-1-yl)-5-pyridyl]-2-(4-morpholino)acetonitril und Crotonsäurenitril 23.1 g 2-(4-Morpholino)-2-[2-(1H-1,2,4-triazol-1-yl)-5-pyridyl]-3-methylglutarsäuredinitril als zähe Masse, woraus nach Hydrolyse und Umsetzung mit Hydrazin-Hydrat 7.4 g der Titelverbindung vom Schmp. 256-57°C aus 85proz. Ethanol erhalten werden.

Nach Beispiel 1 a-c werden folgende Zwischenprodukte erhalten:

6 a) 2-(1H-1,2,4-Triazol-1-yl)-5-cyanopyridin vom Schmp. 210-12°C.

b) 2-(1H-1,2,4-Triazol-1-yl)pyridin-5-aldehyd vom Schmp. 163-66°C.

c) 2-[2-(1H-1,2,4-Triazol-1-yl)-5-pyridyl]-2-(4-morpholino)acetonitril vom Schmp. 167-71°C.

Beispiel 7

4.5-Dihydro-5-methyl-6-[2-(methylaminocarbonylamino)-5-pyridyl]-3(2H)[pyridazinon

Eine Mischung aus 0.4 g (2 mmol) 4,5-Dihydro-5-methyl-6-(2-amino-5-pyridyl)-3(2H)-pyridazinon, 10 ml Dimethylsulfoxid und 0.24 ml (4 mmol) Methylisocyanat läßt man 16 h bei Raumtemperatur rühren, engt im Vakuum ein und verreibt den Rückstand mit Wasser.

Man isoliert 0.25 g Titelverbindung (48 % d.Th.) vom Schmp. 257-259°C.

Das Ausgangsmaterial kann wie folgt hergestellt werden:

a) 9.5 g (80 mmol) 2-Amino-5-cyan-pyridin (J. Heterocycl. Chem. 11, 397 (1974) werden analog Beispiel 1 b reduziert. Man erhält 6.7 g (69 %) 6-Amino-nicotinaldehyd vom Schmp. 143-145°C.

b) Durch Umsetzung der vorgenannten Verbindung mit überschüssigem Acetanhydrid unter Rückfluß isoliert man in 55proz. Ausbeute den N,N-Diacetyl-6-amino-nicotinaldehyd vom Schmp. 130-132°C.

c) Analog Beispiel 1 c wird aus der vorgenannten Stufe das 2-(2-Diacetamido-5-pyridyl)-2-(4-morpholino)-acetonitril in 71 % Ausbeute vom Schmp. 116-118°C erhalten.

d) Analog Beispiel 1 erhält man aus der vorstehenden Verbindung und Crotonsäurenitril und Hydrazinhydrat unter Abspaltung der Acetylgruppen mit 69 % Ausbeute das 4,5-Dihydro-5-methyl-6-(2-amino-5-pyridyl)-3(2H)-pyridazinon vom Schmp. 210-211°C.

Beispiel 8

4,5-Dihydro-5-methyl-6-[2-(acetylamino)-5-pyridyl]-3(2H)-pyridazinon

Zu einer Lösung von 0.6 g (3 mmol) 4,5-Dihydro-5-methyl-6-(2-amino-5-pyridyl)-3(2H)-pyridazinon in 35 ml Methanol tropft man 3.4 ml Acetanhydrid, rührt 2 h bei Raumtemp., filtriert und kristallisiert den Niederschlag aus Methanol um. Man isoliert 0.2 g Titelverbindung (27 % d.Th.) vom Schmp. 230-232°C.

Beispiel 9

4,5-Dihydro-5-methyl-6[2-(1-pyrrolyl)-5-pyridyl]-3(2H)-pyridazinon

Eine Mischung aus 1.0 g (5 mmol) 4,5-Dihydro-5-methyl-6-(2-amino-5-pyridyl)-3(2H)-pyridazinon, 0.66 g (5 mmol) 2,5-Dimethoxy-tetrahydrofuran und 15 ml Essigsäure wird 30 min zum Rückfluß erhitzt. Man engt ein, nimmt den Rückstand in Dichlormethan auf, wäscht mit Natriumhydrogencarbonatlösung, engt ein und chromatographiert an Kieselgel (Elutionsmittel: Trichlorethan/Methanol 19:1). Man erhält 0.5 g der Titelverbindung (39 % d.Th.) vom Schmp. 192-193°C (aus Ether).

Beispiel 10

4,5-Dihydro-5-methyl-6-[2-(phenylamino)-5-pyridyl]-3(2H)-pyridazinon

Analog Beispiel 1 erhält man aus 1.65 g (5.6 mmol) 2-[2-(Phenylamino)-5-pyridyl]-2-(4-morpholino)-acetonitril und Crotonsäurenitril nach Reinigung an Kieselgel (Laufmittel: Dichlormethan/Methanol, 99:1) 1.6 g 2-(4-Morpholino)-2-[2-(phenylamino)-5-pyridyl]-3-methylglutarsäuredinitril als schaumigen Rückstand, woraus nach Hydrolyse, Umsetzung mit Hydrazin-Hydrat und Reinigung an Kieselgel (Laufmittel: Dichlormethan/Methanol, 97:3) 0.6 g der Titelverbindung vom Schmp. 202-204°C aus Ethanol erhalten werden.

Nach Beispiel 1a-c werden folgende Zwischenprodukte erhalten

10 a) 2-Phenylamino-5-cyanopyridin vom Schmp. 180-181°C

10 b) 2-Phenylaminopyridin-5-aldehyd vom Schmp. 148-150°C

10 c) 2-(2-Phenylamino-5-pyridiyl)-2-(4-morhpolino)-acetonitril vom Schmp. 160-161°C

Beispiel 11

In analoger Weise wie in Beispiel 10 beschrieben erhält man:

| | Bezeichnung | Ausb. % | Schmelzpunkt °C (Lsg.mittel) |
|---|---|---|---|
| 1) | 4,5-Dihydro-6-[2-(4-pyridyl-amino)-5-pyridyl]-3(2H)-pyridazinon | 62 | 287-89 $CH_3OH$ |
| 2) | 4,5-Dihydro-5-methyl-[2-(4-pyridylamino)-5-pyridyl]-3-(2H)pyridazinon | 55 | 221-23 ($CH_3OH$) |
| 3) | 4,5-Dihydro-6-[2-(2-thiazolylamino)-5-pyridyl]-3-(2H)-pyridazinon | 48 | 275-77 (EtOH) |

## Ansprüche

1. Pyridazinon-Derivate der allgemeinen Formel I,

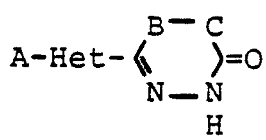

I,

in welcher

Het einen mono-oder bicyclischen Heterocyclus mit ein oder zwei Stickstoffatomen,

B-C eine Gruppe $-CHR_1-CH_2-$, $-CR_1=CH-$, $-CHR_1-O-$, $-O-CHR_1-$, $-CHR_1-NH-$oder $-NH-CHR_1-$, wobei $R_1$ ein Wasserstoffatom oder eine Alkylgruppe bedeutet, und

A eine Amino-, Alkylcarbonylamino-, Aminocarbonylamino-, Aminothiocarbonylamino-, Alkylaminothiocarbonylamino-, Alkylaminocarbonylamino-, N'-Cyano-guanidino-oder N'-Cyano-N"-alkyl-guanidinogruppe, ein ueber ein Stickstoff-oder Schwefelatom gebundener, substituierter Phenylring oder Heterocyclus, oder einen mono-oder bicyclischen, substituierten Heterocyclus mit 1-3 Stickstoffatomen, wobei ein Ringstickstoff direkt an Het gebunden ist, darstellen sowie deren physiologisch verträgliche Salze.

2. Pyridazinon-Derivate der allgemeinen Formel I gemäß Anspruch 1, in welcher

Het einen Pyridin-, Pyrazin-, Pyrimidin-, Pyridazin-oder Phthalazinring bedeutet,

B-C eine Gruppe $-CHR_1-CH_2-$, $-CR_1=CH-$, $-CHR_1-O-$oder $-CHR_1-NH-$darstellt, wobei $R_1$ ein Wasserstoffatom oder eine Methylgruppe ist und

A eine Alkylcarbonylamino-, Aminocarbonylamino-, Aminothiocarbonylamino-, Alkylaminothiocarbonylamino-oder N'-Cyano-N"-alkyl-guanidinogruppe oder einen Amino-, Phenylamino-, Pyridylamino-, Thiazolamino-, Tetrazolamino-oder Triazolthiorest, wobei der Phenylring oder die Heterocyclen durch eine oder mehrere Methyl-, Methoxy-, Hydroxy-oder Chlorreste substituiert sein könne, oder einen Pyrrol-, Pyrazol-, Imidazol-, Imidazolin-, Triazol-, Piperidin-, Piperazin-, Triazin-, Benzimidazol-oder Tetrahydrobenzimidazolrest bedeutet, wobei die Heterocyclen durch eine oder mehrere Methyl-, Hydroxy-, Methylcarbonyl-oder Methylcarbonylaminogruppen substituiert sein können,

sowie deren physiologisch verträglich Salze.

3. Pyridazinon-Derivate der allgemeinen Formel I gemäß Anspruch 1 oder 2, in welcher

Het einen Pyridinring,

B-C eine Gruppe $-CHR_1-CH_2-$oder $-CR_1=CH-$, wobei $R_1$ ein Wasserstoffatom oder eine Methylgruppe bedeutet, und

A einen Alkylcarbonylamino-, Alkylaminocarbonylamino-, Phenylamino-.Pyridylamino.Thiazolamino-.Pyrrol-.Imidazol-.Triazol-, Benzimidazol-oder Tetrahydrobenzimidazolrest bedeuten, sowie deren physiologisch verträgliche Salze.

4. Verfahren zur Herstellung von Pyridazinon-Derivaten der allgemeinen Formel I

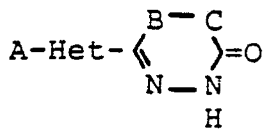

I,

in welcher

Het einen mono-oder bicyclischen Heterocyclus mit ein oder zwei Stickstoffatomen,

B-C eine Gruppe $-CHR_1-CH_2-$, $-CR_1=CH-$, $CHR_1-O-$, $-O-CHR_1-$. $-CHR_1-NH-$oder $-NH-CHR_1-$. wobei $R_1$ eine Wasserstoffatom oder eine Alkylgruppe bedeutet, und

A eine Amino-, Alkylcarbonylamino-, Aminocarbonylamino-, Aminothiocarbonylamino-. Alkylaminothiocarbonylamino-, Alkylaminocarbonylamino-, N'-Cyano-guanidino-oder N'-Cyano-N''-alkyl-guanidinogruppe, ein ueber ein Stickstoff-oder Schwefelatom gebundener, substituierter Phenylring oder Heterocyclus, oder einen mono-oder bicyclischen, substituierten Heterocyclus mit 1-3 Stickstoffatomen. wobei ein Ringstickstoff direkt an Het gebunden ist, darstellen sowie deren physiologisch verträgliche Salze dadurch gekennzeichnet, daß man

a) für den Fall, daß die Gruppe B-C die Gruppe $-CHR_1-CH_2-$oder $-CR_1=CH-$darstellt, γ-Ketocarbonsäuren der allgemeinen Formel II

A-Het-CO-$\overset{R_1}{C}$H-$CH_2$-COOH II,

in der A, Het und $R_1$ die angegebene Bedeutung haben, mit Hydrazin cyclisiert, und gewünschtenfalls anschließend die erhaltenen Verbindungen in Verbindungen mit der Gruppe B-C gleich $-CR_1=CH-$überführt, oder

b) für den Fall, daß die Gruppe B-C die Gruppe $-CHR_1-O-$darstellt, Hydrazone der allgemeinen Formel III

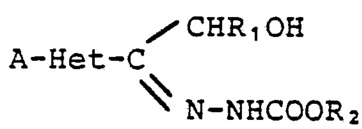

III

in der A, Het und $R_1$ die angegebene Bedeutung haben und $R_2$ einen niederen Alkylrest bedeutet, cyclisiert oder

c) für den fall, daß die Gruppe B-C die Gruppe $-O-CHR_1-$darstellt, Hydrazide der allgemeinen Formel IV

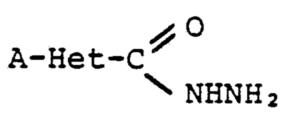

IV,

mit Säuren der allgemeinen Formel V oder reaktiven Derivaten hiervon

Hal-$CHR_1$-COOH V,

in der $R_1$ die angegebene Bedeutung hat und Hal ein Halogenatom darstellt, cyclisiert, oder

d) für den Fall, daß die Gruppe B-C die Gruppe $-NH-CHR_1-$darstellt Iminoether der allgemeinen Formel VI

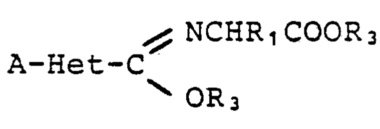

VI,

in der A, Het und $R_1$ die angegebene Bedeutung haben und die beiden Reste $R_3$ gleich oder verschieden sind und Niederalkyl bedeuten, mit Hydrazin cyclisiert oder

e) für den Fall, daß die Gruppe B-C die Gruppe $-CHR_1-NH-$darstellt,

Verbindungen der allgemeinen Formel VII

A-Het = $COCHR_1NHCOOR_4$ VII,

in der A, Het und $R_1$ die angegebene Bedeutung haben und $R_4$ eine Niederalkylrest bedeutet, mit Hydrazin cyclisiert

und anschließend die erhaltene Verbindung der allgemeinen Formel I in üblicher Weise in andere Verbindungen überführt sowie gewünschtenfalls die Verbindungen der Formel I in physiologisch verträgliche Salze überführt.

5. Verfahren zur Herstellung von Pyridazonen-Derivaten der allgemeinen Formel I gemäß Anspruch 4. in welcher

Het einen Pyridin-, Pyrazin-, Pyrimidin-, Pyridazin-oder Phthalazinring bedeutet,

B-C eine Gruppe $-CHR_1-CH_2-$, $-CR_1=CH-$, $-CHR_1-O-$oder $-CHR_1-NH-$darstellt, wobei $R_1$ ein Wasserstoffatom oder eine Methylgruppe ist und

A eine Alkylcarbonylamino-, Aminocarbonylamino-, Aminothiocarbonylamino-, Alkylaminocarbonylamino- oder N'-Cyano-N"-alkyl-guanidinogruppe oder einen Amino-, Phenylamino-, Pyridylamino-, Thiazolamino-, Tetrazolamino-pder

Triazolthiorest, wobei der Phenylring oder die Heterocyclen durch eine oder mehrere Methyl-, Methoxy-, Hydroxy-oder Chlorreste substituiert sein können, oder einen Pyrrol-, Pyrazol-, Imidazol-, Imidazolin-, Triazol-, Piperidin-, Piperazin-, Triazin-, Benzimidazol-oder Tetrahydrobenzimidazolrest bedeutet, wobei die Heterocyclen durch eine oder mehrere Methyl-, Hydroxy-, Methylcarbonyl-oder Methylcarbonylaminogruppen substituiert sein können,

sowie deren physiologisch verträglich Salze.

6. Verfahren zur Herstellung von Pyridazinon-Derivaten der allgemeinen Formel I gemäß Anspruch 4 oder 5,

in welcher

Het einen Pyridinring,

B-C eine Gruppe $-CHR_1-CH_2-$oder $-CR_1=CH-$, wobei $R_1$ ein Wasserstoffatom oder eine Methylgruppe bedeutet, und

A einen Alkylcarbonylamino-, Alkylaminocarbonylamino-, Phenylamino-, Pyridylamino-,Thiazolamino-, Pyrrol-,Imidazol-, Triazol-, Benzimidazol-oder Tetrahydrobenzimidazolrest

bedeuten, sowie deren physiologisch verträgliche Salze.

7. Arzneimittel enthaltend mindestens eine Verbindung gemäß Anspruch 1, 2 oder 3, gegebenenfalls neben üblichen Träger-und/oder Hilfsstoffen.

8. Verbindungen gemäß Anspruch 1, 2 oder 3 zur Herstellung von Arzneimitteln

9. Verwendung von Verbindungen gemäß Anspruch 1, 2 oder 3 zur Herstellung von Arzneimitteln zur Behandlung von Herz-und Kreislauferkrankungen.